(19)

Europäisches Patentamt
European Patent Office
Office européen des brevets

(11) **EP 3 111 850 A1**

(12) **EUROPEAN PATENT APPLICATION**
published in accordance with Art. 153(4) EPC

(43) Date of publication:
**04.01.2017 Bulletin 2017/01**

(21) Application number: **15754706.8**

(22) Date of filing: **19.01.2015**

(51) Int Cl.:
**A61B 8/06** *(2006.01)*  **A61B 8/14** *(2006.01)*

(86) International application number:
**PCT/JP2015/051281**

(87) International publication number:
**WO 2015/129336 (03.09.2015 Gazette 2015/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.02.2014 JP 2014038538**

(71) Applicant: **Hitachi, Ltd.**
**Chiyoda-ku,**
**Tokyo 100-8280 (JP)**

(72) Inventors:
• **TANAKA, Tomohiko**
  **Tokyo 100-8280 (JP)**
• **OKADA, Takashi**
  **Mitaka-shi**
  **Tokyo 181-8622 (JP)**

(74) Representative: **MERH-IP Matias Erny Reichl Hoffmann**
**Patentanwälte PartG mbB**
**Paul-Heyse-Strasse 29**
**80336 München (DE)**

(54) **ULTRASONIC IMAGE PICKUP DEVICE AND METHOD**

(57) Provided is an ultrasound imaging apparatus that is cable of estimating a three-dimensional effect of a blood flow, by using blood-flow velocity information obtained by a color Doppler method, and presenting diagnostic information into which the three-dimensional effect is reflected. The ultrasound imaging apparatus of the present invention is provided with an ultrasound probe configured to transmit ultrasound waves to a test object and to receive echo signals reflected from the test object, and a signal processor configured to process the echo signals received by the ultrasound probe, and the signal processor estimates the three-dimensional effect of the blood flow velocity, from a difference between a first blood flow velocity estimated from the echo signals by a first method, and a second blood flow velocity estimated by a second method which is different from the first method, and generates diagnostic information into which the three-dimensional effect is reflected.

COLOR DOPPLER IMAGE AND MYOCARDIAL TRACKING

FIG. 6

**Description**

**Technical Field**

The present invention relates to an ultrasound imaging apparatus for medical use, and more particularly, to an ultrasound imaging apparatus being capable of estimating a velocity vector of a blood flow, on the basis of information according to the color Doppler method, with a function to estimate a three-dimensional effect of the blood flow velocity.

**Background Art**

According to the color Doppler method, it is only a velocity component in an ultrasound beam direction that can be measured directly. It is not possible to display a flowing direction, that is, in which direction the blood is flowing in a tomographic plane. A method (Vector Flow Mapping: VFM) is suggested, accordingly, where velocity components in an ultrasound beam direction and a direction orthogonal thereto are estimated, using a velocity on a boundary between tissues and the mass conservation law of a two-dimensional flow, and a velocity vector is obtained from the velocity components in the ultrasound beam direction and the orthogonal direction (see Patent Document 1, for example). The VFM is established on the precondition that all the flows are two-dimensional, not having a three-dimensional property, and any flows do not leak out of an imaging area. Therefore, in the VFM, it is not possible to obtain blood flow information in a flowing field like an actual blood that passes through the imaging area. For example, a blood-flow pressure field, or the like, cannot be obtained.

There is known a method of the ultrasound imaging apparatus for performing three-dimensional measurement to obtain a three-dimensional image from an optional point of view (see Patent Document 2). However, it is difficult to estimate three-dimensional properties of a blood flow vector (three-dimensional blood-flow dynamics), on the basis of a three-dimensional image obtained by the three-dimensional measurement.

**Prior Art Document**

**Patent Document**

Patent Document 1 Japanese Unexamined Patent Application Publication No. 2000-342585
Patent Document 2 Japanese Unexamined Patent Application Publication No. 11-313824

**Disclosure of the Invention**

**Problems to be Solved by the Invention**

In order to examine three-dimensional blood flow dynamics in a heart or tumor, three-dimensional blood-flow analysis is important. As described above, however, the VFM intended for the two-dimensional flow has limits, and it is necessary to estimate a three-dimensional effect in a flowing field passing through the imaging area, in addition to the two-dimensional blood flow dynamics within the imaging area.

An object of the present invention is to provide an ultrasound diagnostic apparatus that utilizes blood-flow velocity information obtained by the color Doppler method and estimates a three-dimensional effect of the blood flow, so as to present diagnostic information into which the estimated three-dimensional effect is reflected.

**Means for Solving the Problems**

An ultrasound imaging apparatus of the present invention to solve the problem above, estimates a blood flow velocity using plural methods on the basis of information according to the color Doppler method, further estimates a three-dimensional effect of the blood flow based on consistency of results of the methods, and reflects the three-dimensional effect into diagnostic information.

Namely, the ultrasound imaging apparatus of the present invention is provided with an ultrasound probe configured to transmit ultrasound waves to a test object and to receive echo signals reflected from the test object, and a signal processor configured to process the echo signals received by the ultrasound probe, wherein the signal processor estimates a three-dimensional effect of the blood flow velocity, from a difference between a first blood flow velocity estimated from the echo signals via a first method, and a second blood flow velocity estimated from the echo signals via a second method which is different from the first method, and generates diagnostic information into which the three-dimensional effect is reflected. Here, the "three-dimensional effect" indicates an effect held by a blood-flow velocity component in a direction orthogonal to the two-dimensional space, and includes a spatial rate-of-change of the velocity, which is the blood-flow velocity component, together with various amounts derived therefrom, under the hypothesis of two-dimensional flow, that is, assuming that a blood flow vector to be defined three-dimensionally in actual fact, is defined in the two-dimensional space.

**Advantage of the Invention**

According to the present invention, diagnostic information is provided, which is useful for examining three-dimensional blood flow dynamics.

**Brief Description of the Drawings**

FIG. 1 is an overall block diagram showing an ultrasound imaging apparatus according to an embodiment of the present invention;
FIG. 2 is a flowchart showing operations of a signal processor according to a first embodiment;
FIG. 3(a) illustrates a coordinate system on a tissue image, and FIG. 3(b) illustrates a way to calculate a tissue velocity;
FIG. 4 illustrates a way to calculate a blood flow vector;
FIG. 5 illustrates different ways to calculate the blood flow vector by a different method;
FIG. 6 illustrates a processing in a three-dimensional effect estimator;
FIGs. 7(a) and 7(b) are conceptual diagrams showing conservation of physical quantity;
FIG. 8(a) and 8(b) illustrate how to calculate diagnostic information;
FIG. 9 illustrates operations of the signal processor according to a second embodiment;
FIG. 10 illustrates a display example;
FIG. 11 illustrates a display example;
FIG. 12 illustrates a display example;
FIG. 13 illustrates a display example; and
FIG. 14 illustrates a display example.

**Best Mode for Carrying Out the Invention**

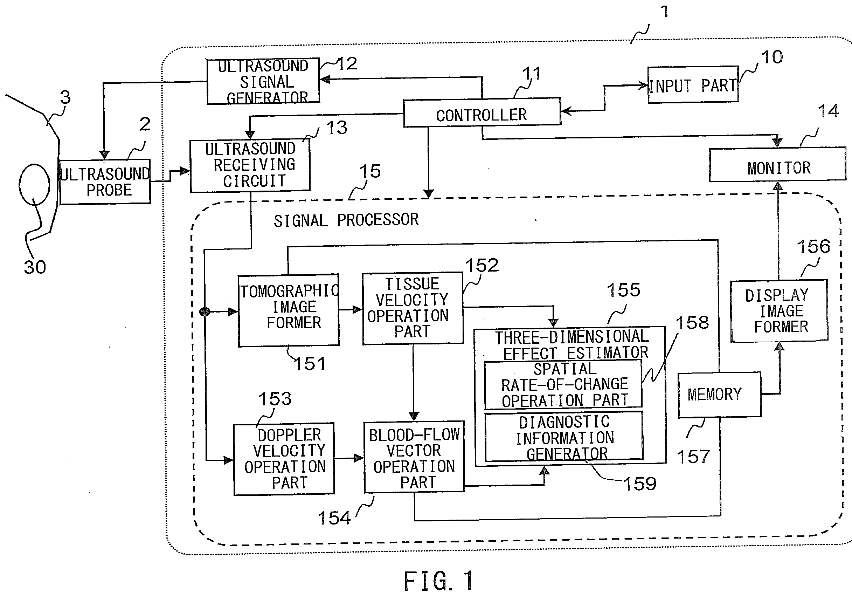

An ultrasound diagnostic apparatus according to embodiments of the invention is provided with an ultrasound probe 2 configured to transmit ultrasound waves to a test object 3 and to receive echo signals reflected from the test object, and a signal processor 15 configured to process the echo signals received by the ultrasound probe, wherein the signal processor 15 is provided with a blood-flow velocity operation part 154 that calculates a blood flow velocity from the echo signals, and a three-dimensional effect estimator 155 that estimates a three-dimensional effect on the basis of the blood flow velocity calculated by the blood-flow velocity operation part.
The blood-flow velocity operation part 154 is provided with a tissue velocity operation part 152 configured to calculate a tissue velocity of the test object, and also provided with a Doppler velocity operation part 153, and calculates the blood flow velocity within an imaging area, by using a tissue and blood-flow boundary velocity calculated by the tissue velocity operation part, and a Doppler velocity calculated by the Doppler velocity operation part.
The three-dimensional effect estimator 155 is provided with a spatial rate-of-change operation part 158 for calculating as the three-dimensional effect, a spatial rate of change of the blood flow velocity in the direction orthogonal to the imaging area, and various amounts derived from the spatial rate of change. It is further provided with a diagnostic information generator 159 that generates diagnostic information by using the three-dimensional effect estimated by the three-dimensional effect estimator.
Embodiments of the present invention will now be described with reference to the accompanying drawings. FIG. 1 is a block diagram showing a configuration example of the ultrasound imaging apparatus according to the present invention, and as shown in FIG. 1, the ultrasound imaging apparatus of the present embodiments includes a main unit 1 and an ultrasound probe 2.
The main unit 1 used for creating an ultrasound image with controlling the ultrasound probe 2, is provided with an input part 10, a controller 11, an ultrasound signal generator 12, an ultrasound receiving circuit 13, a monitor 14, and a signal processor 15.
The ultrasound probe 2 comes into contact with a living body (test subject) 3, irradiates a radiation region 30 with ultrasound waves in accordance with signals generated by the ultrasound signal generator 12, and receives echo signals of reflected waves from the radiation region 30. The ultrasound probe 2 generates continuous waves or pulse waves, depending on a scanning mode.

Constituent elements of the main unit 1 will now be described. The input part 10 is provided with a key board or a pointing device for an examiner who manipulates the ultrasound imaging apparatus to configure settings for the controller 11, with regard to operating conditions of the ultrasound imaging apparatus. The input part also functions as an external signal input part, for the case where information from external equipment such as an ECG, is utilized for the examination.

The controller 11 controls the ultrasound signal generator 12, the ultrasound receiving circuit 13, the monitor 14, and the signal processor 15, according to the operating conditions of the ultrasound imaging apparatus, being set via the input part 10, and the controller may be a CPU of a computer system, for instance.

The ultrasound signal generator 12 is provided with an oscillator for generating signals at predetermined intervals, and transmits drive signals to the ultrasound probe 2. The ultrasound receiving circuit 13 performs signal processing, such as amplification and beamforming, on the reflected echo signals received by the ultrasound probe 2. The ultrasound receiving circuit 13 includes a receiving circuit, an envelope detection means, and a means for performing Log compression. The monitor 14 outputs information that is obtained by the signal processor 15. The signal processor 15 has a function of generating an ultrasound image from the reflected echo signals, received from the ultrasound probe 2. Details of the function above will be described later.

The main unit 1 is further provided with a scan converter and an A/D converter, though not illustrated. The scan converter may be contained in the ultrasound receiving circuit 13, or it may be provided on the subsequent stage of the signal processor 15. When the ultrasound receiving circuit 13 contains the scan converter, there is an advantage that a data amount treated in the signal processor 15 is reduced. On the other hand, when the scan converter is not contained in the ultrasound receiving circuit 13, this allows the signal processor 15 to treat various data, achieving a measuring device with a high degree of precision. The A/D converter is provided in the preceding stage of the signal processor 15. Its sampling frequency may be usually set to be a value from 20 MHz to 50 MHz.

Next, constitutional elements of the signal processor 15 will be described in detail. The signal processor 15 includes, as main components, a tomographic image former 151, a tissue velocity operation part 152, a Doppler velocity operation part 153, a blood-flow vector operation part 154, a three-dimensional effect estimator 155, a display image former 156, and a memory 157.

The tomographic image former 151 may form on the basis of the reflected echo signals outputted from the ultrasound receiving circuit 13, a B-mode image, for example; a two-dimensional tissue form image according to a planar imaging method for imaging an ultrasound irradiation object or a three-dimensional tissue form image according to a stereoscopic imaging method. The tomographic image former 151 may extract tissue location information from the tissue form image. The tissue velocity operation part 152 may extract motion information of the tissue, from the tissue form image. The Doppler velocity operation part 153 may extract, on the basis of the reflected echo signals outputted from the ultrasound receiving circuit 13, blood-flow velocity information of the color Doppler mode, for example; two-dimensional Doppler blood-flow velocity information of the ultrasound irradiation object according to the planar imaging method, or three-dimensional Doppler blood-flow velocity information according to the stereoscopic imaging method. The blood-flow vector operation part 154 may estimate a blood flow vector by using physical laws, on the basis of the Doppler blood-flow velocity information.

The three-dimensional effect estimator 155 may estimate a three-dimensional effect of the blood flow vector, and generate diagnostic information by using this three-dimensional effect. The three-dimensional effect of the blood flow vector indicates an effect held by a blood-flow velocity component in the direction orthogonal to a plane (two-dimensional space) that defines the blood flow vector being calculated by the blood-flow vector operation part 154.

The three-dimensional effect estimator 155 is provided with a spatial rate-of-change operation part 158 for calculating as the three-dimensional effect, a spatial rate of change of the blood flow orthogonal to the imaging area, and various amounts derived therefrom, and a diagnostic information generator 159 for generating diagnostic information by using the three-dimensional effect. Specifically, an arithmetic unit constitutes the three-dimensional effect estimator 155, and execution of programs incorporated in the arithmetic unit may implement functions of the three-dimensional effect estimator 155, including the functions of the spatial rate-of-change operation part 158 and of the diagnostic information generator 159.

The display image former 156 forms a display image to be displayed on the monitor 14, and forms the display image such as a tomographic image formed by the tomographic image former 151, a Doppler waveform obtained by the Doppler measurement, and various amounts calculated by the three-dimensional effect estimator 155, according to a predetermined format or an instruction inputted via the input part 10.

The memory 157 stores reflected echo signals, information necessary for the calculations in the signal processor 15, and a processing result from the signal processor 15.

Considering the configuration of the apparatus as described above, embodiments of the operation of the ultrasound diagnostic apparatus will be described below.

<First Embodiment>

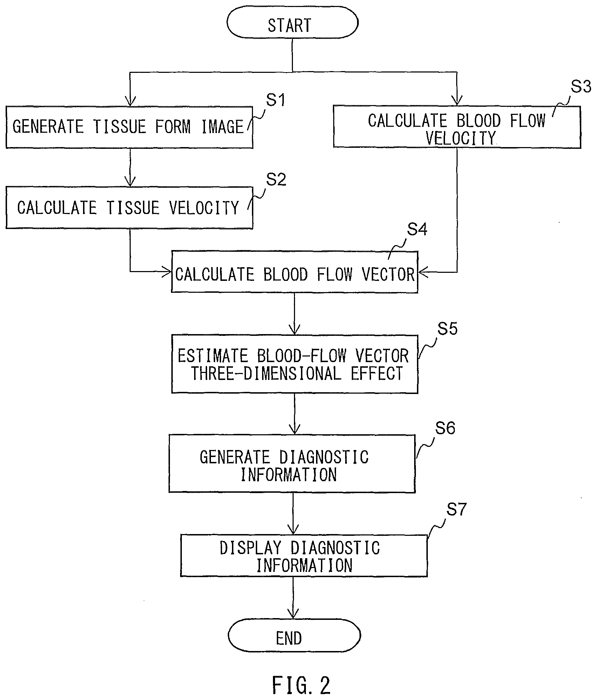

The first embodiment will be described with reference to a processing flowchart as shown in FIG. 2. With reference to FIG. 2, there will be described, as a specific example, the case where a radiation region 30 (FIG. 1) indicates a portion including the left ventricle. However, the radiation region 30 may be a blood vessel or another heart chamber desired by an examiner.

As shown in FIG. 2, in the present embodiment, following processes are performed; the process of forming a tissue form image and calculating a tissue velocity (S1, S2), a process of calculating a blood flow velocity (S3), a process of calculating a blood flow vector by using the tissue velocity and the blood flow velocity (S4), a process of estimating a three-dimensional effect of the blood flow vector by using the blood flow vector (blood flow velocity) calculated by two methods (S5), a process of generating diagnostic information by using thus estimated three-dimensional effect (S6), and the process of displaying the diagnostic information (S7). It is alternatively possible to omit the process of generating the diagnostic information, and to perform the process of displaying the three-dimensional effect that is estimated in the step of S7. There will now be described details of the processes herein below.

<Step S1>

Firstly, in order to calculate a tissue velocity in the radiation region (left ventricle), an image is taken for obtaining morphological information (B-mode image) of the radiation region. An ultrasonic frequency of the B-mode image is assumed to fall into a range from 1 MHz to 20 MHz which enables imaging. A frame rate is assumed to fall into a range that allows capturing of cardiac movement that varies depending on heart beats. Specifically, it is assumed as equal to or higher than 15 Hz. The tomographic image former 151 may form the B-mode image, for example, on the basis of the reflected echoes outputted from the ultrasound receiving circuit 13. The ultrasound biological image may be any of a two-dimensional image using a planar imaging method, and a three-dimensional image using a stereoscopic imaging method, and data is acquired in time series.

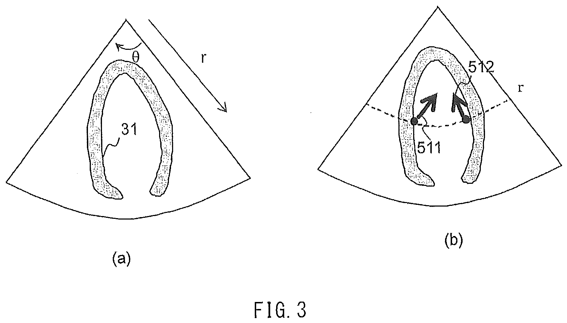

FIG. 3(a) illustrates one example of the morphological information obtained by the step S1. FIG. 3 shows the case where a sector probe that performs sector scanning is used as the ultrasound probe 2 and the left ventricle 31 is set as an imaging target. In the figures, r indicates a beam direction (depth direction) of ultrasound waves, and θ indicates a beam angle direction within the imaging area. For the case of sector scanning, the r-direction is assumed as the depth direction, and the θ-direction is assumed as the scanning direction.

<Step S2>

The tissue velocity operation part 152 firstly acquires tissue positional information, from the ultrasound biological image that is formed by the tomographic image former 151 in the step S1. The tissue positional information may be detected by subjecting an inner wall of tissue to image processing, or the examiner may designate the inner wall of tissue via the input part 10 to acquire the positional information. Specifically, since the tissue is recognized as a high-intensity value in the ultrasound image, a portion with the high-intensity value is assumed as cardiac tissue, and accordingly, a two-dimensional or three-dimensional cardiac tissue location is acquired. Alternatively, the examiner may designate an inner wall being a boundary surface between blood and tissue via a pointing device provided on the input part 10, thereby providing positional information. Those methods above are collectively referred to as myocardial tracking.

Next, the tissue velocity operation part 152 calculates a tissue and blood-flow boundary velocity. The tissue and blood-flow boundary velocity indicates a velocity on the boundary between the tissue wall and blood, and hydrodynamically, a velocity of the blood is equal to a velocity of tissue on this boundary surface. As a method for calculating the tissue and blood-flow boundary velocity, a pattern matching of two time-series images may be employed, or it is possible to trace a temporal development of the tissue positional information which is determined as described above, in other words, the movement of tissue may be traced. As a calculation method of the pattern matching, for example, a cross correlation method, SAD (sum of absolute difference) method, SSD (sum of squared difference) method, or KLT (Kanade-Lucas-Tomai) method may be employed. A shift amount of the tissue obtained by those methods is divided by an imaging interval, thereby calculating the tissue and blood-flow boundary velocity.

As the tissue and blood-flow boundary velocity, as shown in FIG. 3(b) for example, the tissue and blood-flow boundary velocity 511 of the left-side tissue and the tissue and blood-flow boundary velocity 512 of the right-side tissue on the same depth, are calculated.

<Step S3>

In addition to the imaging for obtaining the morphological information in the step S1, measurement according to a Doppler method is performed on the radiation region that corresponds to the radiation region imaged in the step 1, and blood-flow velocity distribution information is obtained. The Doppler method may be either of a continuous-wave Doppler method and a pulse-wave Doppler method. In this example here, a color Doppler method, being a versatile method, is employed. In this case, the Doppler velocity operation part 153 focuses on a blood portion within the ultrasound biological image that is acquired by the tomographic image former 151, and the blood-flow velocity distribution information is acquired by an auto-correlation method. The blood -flow velocity distribution obtained here may be a component of the blood flow velocity, in the beam direction (r-direction) of the ultrasound beam.

<Step S4>

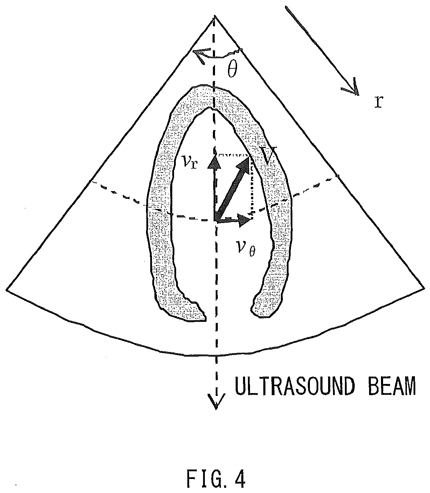

The blood-flow vector operation part 154 estimates a blood flow vector, by using the tissue and blood-flow boundary velocity calculated by the tissue velocity operation part 152, and the blood-flow velocity distribution information acquired by the Doppler velocity operation part 153. With reference to FIG. 4, a method of estimating the blood flow vector will be described. A velocity of the blood flow passing through the imaging area has a three-dimensional velocity component. With the velocity measurement using the Doppler effect, only a velocity component in the ultrasound beam direction can be obtained, out of the three-dimensional velocity components. However, use of a physical law (a law of conservation of mass) enables estimation of a velocity component in the beam perpendicular direction.
Specifically, a continuity equation representing the law of conservation of fluid mass in a polar coordinate system is described as the formula 1.
[Formula 1]

$$\frac{v_r}{r} + \frac{\partial v_r}{\partial r} + \frac{1}{r}\frac{\partial v_\theta}{\partial \theta} + \frac{\partial v_z}{\partial z} = 0 \qquad (1)$$

where r is a depth direction of the ultrasound beam, $\theta$ is a sector-scanning direction of the ultrasound beam, and $v_r$, $v_\theta$, and $v_z$ are velocity components of the blood flow, respectively, in the r-direction, in the $\theta$-direction, and in the direction perpendicular to the imaging area.
When influence of the blood flow velocity $v_z$ passing perpendicularly through the imaging area is ignored, the mass conservation equation 1 is represented by the following formulas 2 and 3:
[Formula 2]

$$\frac{v_r}{r} + \frac{\partial v_r}{\partial r} + \frac{1}{r}\frac{\partial v_\theta}{\partial \theta} = 0 \qquad (2)$$

[Formula 3]

$$\frac{\partial v_z}{\partial z} = 0 \qquad (3)$$

The formula 2 may be replaced by the formula 4. [Formula 4]

$$\frac{\partial v_\theta}{\partial \theta} = -v_r - r\frac{\partial v_r}{\partial r} \qquad (4)$$

According to the formula 4, the blood flow velocity in the $\theta$-direction $v_\theta(r, \theta)$ can be obtained by the formula 5.
[Formula 5]

$$v_{\theta}(r,\theta) = v_{\theta r}(r) + \int \left( -v_r(r,\theta) - r\frac{\partial v_r(r,\theta)}{\partial r} \right) d\theta \qquad (5)$$

In the formula 5, $v_{\theta 0}(r)$ is a velocity component of the tissue and blood-flow boundary velocity on the depth r in the direction orthogonal to the beam direction, which is calculated by the tissue velocity operation part 152. Integration of the second term on the right side of the formula 5 corresponds to the integration in the $\theta$-direction on the depth r.

As shown in FIG. 4, the velocity vector $V(r, \theta)$ in the imaging area can be obtained, from the velocity component $v_\theta(r, \theta)$ in the direction orthogonal to the beam obtained by the formula 5, and the velocity component $v_r(r, \theta)$ in the ultrasound beam direction obtained by the color Doppler method. In FIG. 4, only one-point vector V is shown, but vectors are calculated on plural points included in a desired area being the VFM target.

As shown in formula 3, the velocity vector V calculated as described above does not include the influence of the blood flow perpendicularly passing through the imaging area. Therefore, in the next step, the blood-flow vector operation part 154 calculates the velocity vector V according to a different approach, with regard to the same position, in order to estimate the influence of the blood flow perpendicularly passing through the imaging area, i.e., the three-dimensional effect of the blood flow vector.

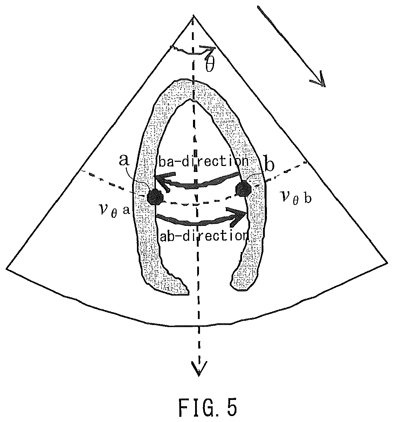

As shown in FIG. 5, by way of example, when the position is assumed from the point a to the point b of the tissue and blood-flow boundary (right and left myocardium), there are employed two methods; a method of calculating a velocity vector by integrating from left to right (the first method), and a method of calculating the velocity vector by integrating from right to left (the second method).

In the first method, the formula 5 is replaced by the formula 6.

[Formula 6]

$$v_{\theta}^{(1)}(r,\theta) = v_{\theta a}^{T}(r) + \int_{a}^{\theta} \left( -v_r(r,\theta) - r\frac{\partial v_r(r,\theta)}{\partial r} \right) d\theta \qquad (6)$$

where the velocity in the first term on the right side in the formula 6 is a velocity component in the $\theta$-direction calculated by the tissue velocity operation part 152, with regard to the point a determined by the myocardial tracking.

In the second method, the formula 5 is replaced by the formula 7.

[Formula 7]

$$v_{\theta}^{(2)}(r,\theta) = v_{\theta b}^{T}(r) - \int_{\theta}^{b} \left( -v_r(r,\theta) - r\frac{\partial v_r(r,\theta)}{\partial r} \right) d\theta \qquad (7)$$

where the velocity in the first term on the right side in the formula 7 is a velocity component in the $\theta$-direction calculated by the tissue velocity operation part 152, with regard to the point b determined by the myocardial tracking.

<Step S5>

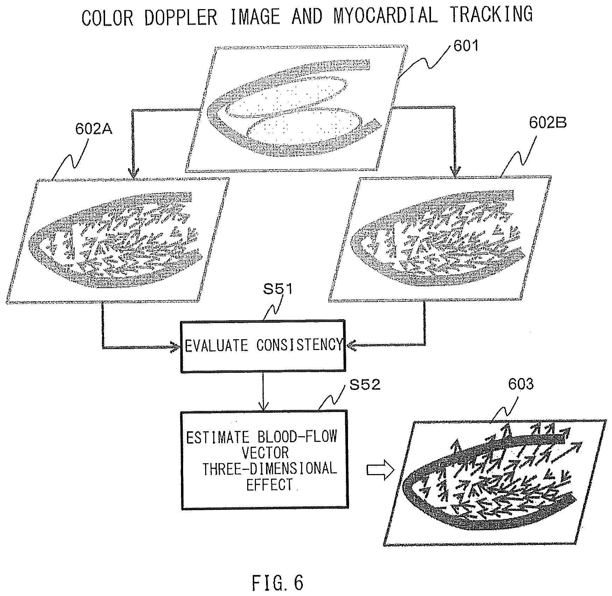

In the step S4, the three-dimensional effect estimator 155 uses the blood flow velocities being the results calculated by the two methods, firstly evaluates consistency of the results, and then, estimates a three-dimensional flow effect of the blood-flow vector on the basis of thus evaluated consistency. FIG. 6 shows details of the estimating process of the three-dimensional effect estimator 155.

As shown in FIG. 6, there is obtained the tissue and blood-flow boundary information 601 determined by the color Doppler image and the myocardial tracking in the steps S1 to S3. In the step S4, the blood flow vector fields 602A and 602B obtained by two methods are calculated. Those blood flow vector fields 602A and 602B are obtained under the hypothesis of two-dimensional flow on the imaging section.

As shown in the formula 8, the three-dimensional effect estimator 155 calculates a difference between the blood flow vectors obtained by the two methods, and evaluates consistency thereof (S51):

[Formula 8]

$$D(r) \equiv v_\theta^{(2)}(r,\theta) - v_\theta^{(1)}(r,\theta) \qquad (8)$$

It should be noted that D(r) obtained by the formula 8 is a function of depth, since it is angle-independent and it remains constant along the integral path. The angle $\theta$ upon calculating D (r) may be any value as far as it is within the VFM target area, and it is able to be calculated at any point on the integral path, without being limited to the tissue blood boundary. It should be noted that in the formula 8, the blood-flow velocity component $v\theta$ in the $\theta$-direction is used as the blood-flow vector field for obtaining a difference. It is also possible to use the blood flow vector V which is obtained by the velocity component $v_\theta$ in the $\theta$-direction and the velocity component $v_r$ in the beam direction.

When the measurement object is a two-dimensional flow, the formula 9 is established, and D(r) in the formula (8) becomes zero.

[Formula 9]

$$v_\theta^{(1)}(r,\theta) = v_\theta^{(2)}(r,\theta) \qquad (9)$$

However, since a flow such as the flow in the left ventricle of the heart is three-dimensional, the formula 3 is not viable, to be exact. The formula 8 is not viable either, due to the same reason.

In this situation, a blood-flow vector three-dimensional effect 603 is estimated by using D(r) ($\neq$ 0) (S52). The blood flow vector calculated in the step S4 may include various errors such as errors in measurement precision, in addition to errors caused by the blood-flow vector three-dimensional effect. Many of the errors, other than the errors caused by the blood-flow vector three-dimensional effect, may be included in both of the blood flow vectors used for subtraction, with the same magnitude and the same sign, respectively, and thus such errors are removed by the subtraction. Therefore, a main factor that hampers D(r) from becoming zero may be considered as failure under the hypothesis of two-dimensional flow.

On the precondition above, as shown in the formula 10, the three-dimensional effect estimator 155 defines D(r) as a summation of blood-flow vector three-dimensional effects of discretized individual blood vectors on the integral path, and individual three-dimensional effects are calculated.

[Formula 10]

$$D(r) \equiv r\Delta\theta \sum_{i=1}^{N} \frac{\partial v_z}{\partial z}\bigg|_i \qquad (10)$$

where $\dfrac{\partial v_z}{\partial z}\bigg|_i$ is a spatial rate of change of velocity in the direction passing through the imaging area, with regard to the i-th blood flow vector on the integral path

In the formula 10, N indicates the number of the blood flow vectors on a certain depth r on the integral path, and $\Delta\theta$ indicates a beam angle interval. It is to be noted that the number N of the blood flow vectors is one of various amounts that depend on the number of ultrasound beams, and it can also be called as the sampling points on the integral path.

Since D(r) indicates a summation on the integral path, individual three-dimensional effects cannot be derived from the formula 10. The three-dimensional effect estimator 155 (spatial rate-of-change of velocity operation part 158) configures a distribution model of the spatial rate of change of velocity as to each blood flow vector, and estimates from D(r), the spatial rates of change of velocity with regard to individual blood flow vectors. Any distribution model is applicable as far as it complies with physical laws. Some model examples and a method of calculating the spatial rate of change of velocity using those examples will be described herein below.

As the simplest case, there is a model example where the spatial rate of change of velocity in the direction perpendicular to the imaging area is uniform on the integral path. With this model, the individual spatial rates of change of velocity (individual sampling points) are expressed by the formula 11.

[Formula 11]

$$\frac{\partial v_z}{\partial z} = \frac{D(r)}{Nr\Delta\theta} \qquad (1\,1)$$

As a second model, as expressed by the formula 12, there is shown a model where the spatial rate of change of velocity is proportional to the velocity in the beam direction. This model presupposes that as the velocity becomes higher, there is a higher possibility that fluid passes through the imaging area.
[Formula 12]

$$\frac{\partial v_z}{\partial z} = c_2 v_r \qquad (1\,2)$$

where $c_2$ is a proportional constant. When the right side of the formula 12 is assigned into the formula 10, the formula 13 is obtained. Then, the right side of the formula 13 is assigned into the formula 12, then individual spatial rates of change are obtained according to the formula 14.
[Formula 13]

$$c_2 = \frac{D(r)}{r\Delta\theta\sum_{i=1}^{N}(v_r)_i} \qquad (1\,3)$$

[Formula 14]

$$\frac{\partial v_z}{\partial z} = \frac{D(r)}{r\Delta\theta\sum_{i=1}^{N}(v_r)_i} v_r \qquad (1\,4)$$

As a third model, as expressed by the formula 15, there is shown a model where the spatial rate of change of velocity in the direction perpendicular to the imaging area, is proportional to the spatial rate of change of velocity in the beam direction. This model also presupposes that as the velocity becomes higher, there is a higher possibility that fluid passes through the imaging area.
[Formula 15]

$$\frac{\partial v_z}{\partial z} = c_3 \frac{\partial v_r}{\partial r} \qquad (1\,5)$$

where $c_3$ is a proportional constant.
Similar to the second model, individual spatial rates of change are obtained by the formula 16 that is obtained by assigning the right side of the formula 15 into the formula 12.
[Formula 16]

$$\frac{\partial v_z}{\partial z} = \frac{D(r)}{r\Delta\theta\sum_{i=1}^{N}\left(\frac{\partial v_r}{\partial r}\right)_i} \frac{\partial v_r}{\partial r} \qquad (1\,6)$$

It is to be noted that the second model and the third model are based on that the spatial rate of change of velocity is proportional to the velocity in the beam direction, or proportional to the spatial rate of change of velocity in the beam direction. Alternatively, those models may be based on a relationship with a blood flow velocity in the perpendicular beam direction, absolute blood flow velocity, the spatial rate of change of velocity in the beam direction, or the absolute value thereof.

As a fourth model, an example of the model is expressed by the formula 17, where the spatial rate of change of velocity in the direction perpendicular to the imaging area depends on a distance from a heart wall. It is presumed here that as the distance from the heart wall becomes longer, the spatial rate of change of velocity becomes higher. This relationship may not be necessarily proportional, but it may be inversely proportional.

[Formula 17]

$$\left.\frac{\partial v_z}{\partial z}\right|_i = c_4 \left( \frac{N}{2} - \left| i - \frac{N}{2} \right| \right) \qquad (17)$$

where N is identical to N that is defined in the formula 10.

The formula 18 expresses individual spatial rates of change for the case where the above model is employed:

[Formula 18]

$$\frac{\partial v_z}{\partial z} = \frac{D(r)}{r \Delta\theta \sum\limits_{i=1}^{N} \left( \frac{N}{2} - \left| i - \frac{N}{2} \right| \right)} \left( \frac{N}{2} - \left| i - \frac{N}{2} \right| \right) \qquad (18)$$

There has been described calculation of individual three-dimensional effects on the integral path, by using D (r) at a certain depth r on the integral path. Performing this calculation on various depths allows estimation of the spatial rate of change of velocity in the perpendicular direction of the imaging area, within the all desired VFM regions (within a left ventricle). In other words, it is possible to obtain a map of the spatial rates of change of velocity in association with the VFM.

The three-dimensional effect estimator 155 may map spatially thus estimated spatial rates of change of velocity, or alternatively, another index may be created. By way of example, based on the reflected echo signals measured with time, three-dimensional effects may be obtained in time series, and a time change of the three-dimensional effect (spatial rate of change of velocity) on a focused portion may be calculated. Alternatively, in order to show spatial variation of the spatial rate of change of velocity in the imaging area, a ratio, a percentage, or a combination thereof, may be calculated with respect to the spatial rate of change of velocity at a predetermined position. In addition, the three-dimensional effects in time series, or three-dimensional effects obtained at different timing are stored, and a maximum three-dimensional effect of the blood flow vector (maximum value), a minimum three-dimensional effect of the blood flow vector (minimum value), an average three-dimensional effect of the blood flow vector (average value) may be calculated.

Those various amounts may be utilized as indexes to estimate the state where flowing is accelerated or decelerated, in the direction perpendicular to the imaging area.

<Step S6>

The three-dimensional effect estimator 155 (diagnostic information generator 159) uses the three-dimensional effect calculated in the step S5, i. e. , a space distribution of velocity in the direction orthogonal to the imaging area, so as to correct the diagnostic information calculated under the hypothesis of two-dimensional flow, or to generate new diagnostic information.

As an example of the diagnostic information, calculation of pressure gradient will be described. In general, there are two methods for calculating a pressure distribution of incompressible fluid like a blood flow; a method employing the Navier-Stokes equations (NSE method) and a method employing the Pressure Poisson equations (PPE method). The PPE method will be described herein below, having advantages that it is low in calculation cost relative to the NSE method, and by entering sufficient boundary conditions, a pressure field can be obtained without the necessity of time information. For ease of explanation, a Cartesian coordinate system is employed, but as a matter of course, a polar coordinate system may be used as well.

The equation of PPE method is described as the formula 19, in the Tensor notation.

[Formula 19]

$$\frac{1}{\rho}\frac{\partial^2 p}{\partial x_1 \partial x_1} + \frac{1}{\rho}\frac{\partial^2 p}{\partial x_2 \partial x_2} + \frac{1}{\rho}\frac{\partial^2 p}{\partial x_3 \partial x_3} = -\left(\frac{\partial u_1}{\partial x_1}\right)^2 - \left(\frac{\partial u_2}{\partial x_2}\right)^2 - \left(\frac{\partial u_3}{\partial x_3}\right)^2$$

$$-2\frac{\partial u_1}{\partial x_2}\frac{\partial u_2}{\partial x_1} - 2\frac{\partial u_1}{\partial x_3}\frac{\partial u_3}{\partial x_1} - 2\frac{\partial u_2}{\partial x_3}\frac{\partial u_3}{\partial x_2} \qquad (19)$$

where p is pressure, u is a velocity, subscripts represent directions; 1 is depth direction, 2 is a direction orthogonal to the direction of 1 within the imaging area, and 3 is a direction perpendicular to the imaging area (hereinafter the same).

Since the blood flow vector calculated in the step S4 is obtained by the two-dimensional measurement, it is not possible to acquire all the terms in the formula 19. In the case of the two-dimensional measurement on the presumption that there is no influence of a distortion rate toward the out-of-plane direction, the formula 19 becomes the formula 20.

[Formula 20]

$$\frac{1}{\rho}\frac{\partial^2 p}{\partial x_1 \partial x_1} + \frac{1}{\rho}\frac{\partial^2 p}{\partial x_2 \partial x_2} = -\left(\frac{\partial u_1}{\partial x_1}\right)^2 - \left(\frac{\partial u_2}{\partial x_2}\right)^2 - 2\frac{\partial u_1}{\partial x_2}\frac{\partial u_2}{\partial x_1} \qquad (20)$$

Here, the formula 20 is a simplified equation, which does not include sufficient information. By way of example, in the case where there is an inflow during extremely short period, such as an early left-ventricular diastolic phase, velocity variation in passing through the imaging area has a great impact, and it is difficult to represent the situation only by the PPE method as expressed in the formula 20. However, in the PPE method, employing a three-dimensional vector field with sufficient spatial and temporal resolution may enable reflection of a temporal acceleration term that is obtained according to the boundary condition between cross sections, thereby enhancing information precision. In the present embodiment, a three-dimensional effect of the blood flow vector calculated in the step S5, assumed as a correction term C, is added as shown in the formula 21, so as to improve precision in the pressure distribution calculated by the PPE method.

[Formula 21]

$$\frac{1}{\rho}\frac{\partial^2 p}{\partial x_1 \partial x_1} + \frac{1}{\rho}\frac{\partial^2 p}{\partial x_2 \partial x_2} = -\left(\frac{\partial u_1}{\partial x_1}\right)^2 - \left(\frac{\partial u_2}{\partial x_2}\right)^2 - 2\frac{\partial u_1}{\partial x_2}\frac{\partial u_2}{\partial x_1} + C \qquad (21)$$

where C is expressed by the formula 22.

[Formula 22]

$$C = \frac{\partial}{\partial t}\left(\frac{\partial u_3}{\partial x_3}\right) + u_1 \frac{\partial}{\partial x_1}\left(\frac{\partial u_3}{\partial x_3}\right) + u_2 \frac{\partial}{\partial x_2}\left(\frac{\partial u_3}{\partial x_3}\right) \qquad (22)$$

 As seen from the formula 22, C consists of terms representing time derivative and space derivative of the spatial rate of change of velocity in the direction orthogonal to the imaging area (two-dimensional plane) calculated in the step S5. Those terms can be calculated by retrieving temporal information from temporal and spatial calculation results obtained by the three-dimensional effect estimator 155, being stored in the memory 157.

A solution method of the formula 21 is similar to that of the formula 20 according to the PPE method, and the formula 21 is discretized at each point of the directive components of the blood flow vectors, thereby establishing equations of thus discretized formula 21. In addition, it is possible to establish simultaneous equations the number of which is equivalent to the number of vectors, and to obtain solutions from the equations assumed as inverse problems. There are various methods of discretization, such as central difference, forward difference, and backward difference, and a method employing staggered grid. It is not particularly limited, but using the staggered grid is preferable, being the most typical method. As a method for solving the inverse problem, there exist various publicly-known solutions, and thus specific descriptions thereof will not be given here.

As the boundary condition, Neumann boundary condition of differential type, or Dirichlet boundary condition where a numerical value is given, may be applicable. Pressure gradient information obtained from Navier-Stoke equations may be added to the boundary condition. Since those techniques are publicly known, details thereof will not be described here.

In the PPE method, it is necessary to designate reference pressure at one point when the pressure distribution is calculated. As described in the present embodiment, when the left ventricle is targeted, the examiner is allowed to designate the reference pressure at a desired portion, such as an area around a heart apex, a heart base, and an area inside the left atrium. The reference pressure may be a value measured by a measurement method that is different from the ultrasound imaging, and it is settable via the input part 10.

As described above, adding to the PPE method, the three-dimensional effect represented by the correction term, enables proper reflection of impact that is caused by velocity variation in passing through the imaging area, whereby a more precise pressure distribution can be obtained.

The correction term C is not limited to the aforementioned example. It may be a velocity field or a velocity derivative, or a combination thereof, which includes out-of-plane impact and which can be obtained from two-dimensional information. In addition, simplification will be performed as appropriate.

In addition, considering the law of conservation of momentum in the out-of-plane direction may enable estimation of a strain component of the out-of-plane velocity. In this case, the formula with the correction term being added, may be described as the following formula 23:

[Formula 23]

$$\frac{1}{\rho}\frac{\partial^2 p}{\partial x_1 \partial x_1} + \frac{1}{\rho}\frac{\partial^2 p}{\partial x_2 \partial x_2} = -2\frac{\partial}{\partial t}\left(\frac{\partial u_3}{\partial x_3}\right) - 2u_1\frac{\partial}{\partial x_1}\left(\frac{\partial u_3}{\partial x_3}\right) - 2u_2\frac{\partial}{\partial x_2}\left(\frac{\partial u_3}{\partial x_3}\right)$$
$$-\frac{\partial u_2}{\partial x_1}\frac{\partial u_1}{\partial x_2} - \frac{\partial u_1}{\partial x_2}\frac{\partial u_2}{\partial x_1} + 2\frac{\partial u_1}{\partial x_1}\frac{\partial u_2}{\partial x_2} \qquad\qquad (2\ 3)$$

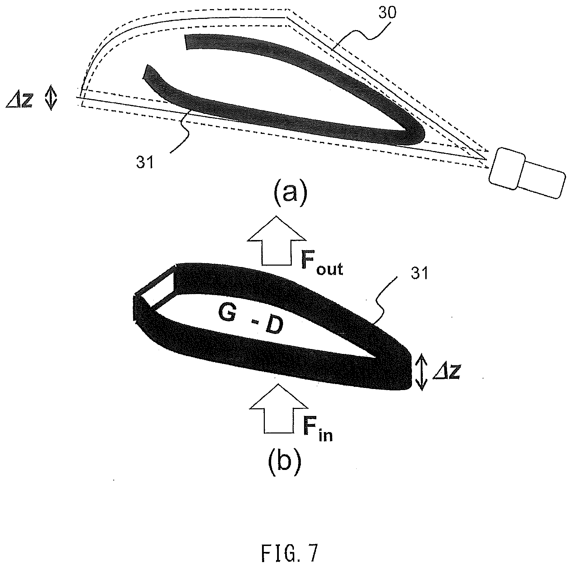

Next, as another example of the diagnostic information generated by the three-dimensional effect estimator 155, a method of estimating a flux in a control volume to be imaged will be described, with reference to FIG. 7. The flux is a unknown value when the law of conservation of physical quantity is applied to the control volume, and the three-dimensional effect estimator 155 estimates the flux by using the three-dimensional effect that is estimated by the step S5.

FIG. 7 (a) illustrates the way how a planar image of a heart is taken, and an image of the left ventricle is taken within the radiation region 30. In here, an example of two-dimensional planar imaging is shown for ease of explanation, but even in the case of planar imaging, there exists a thickness Δz in the imaged plane as indicated by the dotted line. Therefore, the imaging region with the thickness corresponds to the control volume as a target for estimating the flux.

When focusing on a predetermined physical quantity A in this volume, the law of conservation of the physical quantity A is determined in general as shown in FIG. 7(b), by generation G and dissipation D of the physical quantity A in the control volume, inflow flux $F_{in}$ into the control volume, outflow flux $F_{out}$, and an external cause E that depends on the physical quantity. A term necessary for the law of conservation is a remaining amount (flux) of the physical quantity A, being a difference between the inflow flux $F_{in}$ and outflow flux $F_{out}$. The diagnostic information generator 159 uses the spatial rate of change of velocity as to the blood flow passing through the slice-like control volume, thereby calculating the flux of the physical quantity. A method of the calculation will be described below, together with a specific example of the physical quantity.

The thickness Δz of the control volume, necessary for the calculation, is a thickness of the radiation region, i.e., a beam width in the thickness direction. This thickness may be determined by an irradiation position, or it may be a maximum, a minimum, or an average beam width. Biological density $\rho$ necessary for the calculation is a constant between or equal to 1,000 and 1,100 kg/m$^3$, and values for respective tissues are known by document. Representative values may be selected from such document values for respective tissues. Similarly, as for viscosity, a representative value may be selected from those document values.

The physical quantity A is an optional quantity, and it may be any of mass, momentum, kinetic energy, circulation, a quantity of heat, a concentration of a material such as a contrast medium, and the like.

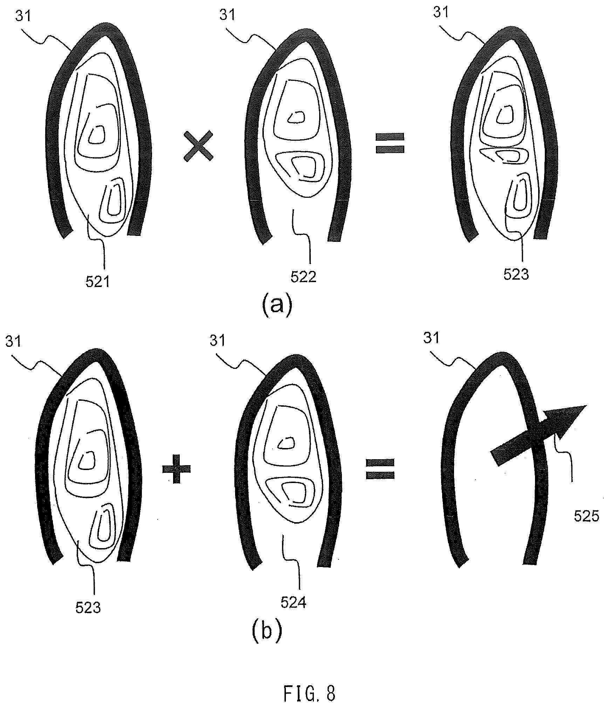

With reference to FIG. 8(a), a calculation example will now be described, regarding the remaining amount of the kinetic energy, as an example of the physical quantity A. The kinetic energy K can be obtained according to the formula 24, by using the blood flow vector information $u_1$ and $u_2$ on each of grid points (cross points between sampled rows in the x-direction and sampled columns in the y-direction on the orthogonal coordinate).

[Formula 24]

$$K = \frac{1}{2}\left(u_1^2 + u_2^2\right) \qquad (24)$$

The distribution chart 521 as shown in the left side of FIG. 8 (a) is a distribution chart of the kinetic energy thus obtained. As shown in FIG. 8 (a), a product of the kinetic energy 521 and the spatial rate of change of velocity 522 in the z-direction (a direction orthogonal to the imaging area) calculated in the step S5 is obtained with respect to each calculation point (grid point), thereby obtaining the remaining amount 523 of the kinetic energy. Specifically, the remaining amount (flux) F of the kinetic energy can be obtained according to the next formula 25.

[Formula 25]

$$F = K\frac{\partial v_z}{\partial z} \qquad (25)$$

Furthermore, as shown in the formula 26, since the generation G and dissipation E of the kinetic energy can be calculated by using the velocity vector information being measured, it is possible to investigate details of the law of conservation of kinetic energy within the control volume, and to check an impact on the myocardial burden upon ejecting blood flow from the heart, and an ejection efficiency of the heart. It is a matter of course that any of the terms in the equation of the law of energy conservation may be focused on.

[Formula 26]

$$\frac{dK}{dt} = G + F - E \qquad (26)$$

As another example of the physical quantity A, a calculation example of momentum will be described with reference to FIG. 8(a) and FIG. 8(b), which are referred to in calculating the kinetic energy. It is to be noted that in FIG. 8(a) here, the distribution chart 521 is read as a distribution chart of the momentum, and the remaining amount 523 is read as a remaining amount of the momentum. Similar to the case of the kinetic energy, a planar distribution chart of the momentum in the x-direction or in the y-direction is able to be calculated by using the velocity vector information. In the case of the momentum, the distribution corresponds to the vector information. Similar to the case of the kinetic energy, a product of the spatial rate of change of velocity 522 in the z-direction calculated in the step S5 and the momentum distribution 521 is obtained with respect to each calculation point, thereby obtaining the remaining amount. In addition, as shown in FIG. 8(b), the sum of a temporal change distribution of the momentum 524 and the remaining amount 523 is obtained, and a total sum thereof is further obtained, thereby calculating a tissue blood-flow interaction force 525.

A method of the calculation above will be described specifically in the following. The momentum M held by individual vectors is expressed by the formula 27, by using a biological density $\rho$ and a micro control volume V held by a grid. The volume V held by the grid can be calculated, assuming the micro control volume as a cubic, for instance, by using lattice spacing in the x-direction and in the y-direction, and a beam thickness.

[Formula 27]

$$\overrightarrow{M} = \rho V \begin{pmatrix} u_1 \\ u_2 \end{pmatrix} \qquad (27)$$

Momentum within a desired region in the heart, for example, within the left ventricle, may be calculated by designating a desired region separately by the examiner with the use of a pointing device, or by utilizing all over the VFM calculation area.

Temporal change of the sum of momentum as to the whole desired region and the momentum flux are equal to the tissue blood-flow interaction force being the sum of force applied to the desired region. Namely, the relationship as expressed by the formula 28 is established.

[Formula 28]

$$\frac{d\sum \overrightarrow{M}}{dt} + \overrightarrow{F_M} = \overrightarrow{f} \qquad (28)$$

where $\dfrac{d\sum \overrightarrow{M}}{dt}$ is temporal change of sum of momentum, $\overrightarrow{F_M}$ is momentum flux, and $\overrightarrow{f}$ is tissue blood-flow interactive force

The temporal change of the sum of momentum can be calculated from time-dependent VFM. The momentum flux can be obtained from the formula 29.

[Formula 29]

$$\overrightarrow{F_M} = \overrightarrow{M}\frac{\partial v_z}{\partial z} \qquad (29)$$

By using the formulas 27 to 29, a vector of the tissue blood-flow interaction force (FIG. 8: 525*) can be obtained.

It is to be noted that the tissue blood-flow interaction force is a vector, and there are calculated an x-direction component of the tissue blood-flow interaction force, a y-direction component of the tissue blood-flow interaction force, and a combined vector of the tissue blood-flow interaction force, respectively obtained by calculating a conservation of momentum only in the x-direction, a conservation of momentum only in the y-direction, and a conservation of momentum in both the x and y directions. Thus obtained directions of the tissue blood-flow interaction force are expected to be useful for diagnosis or asynchronous diagnosis of a torsional motion of the heart.

<Step S7>

The aforementioned diagnostic information estimated by the three-dimensional effect estimator 155, is made into a display image generated by the display image generator 156, including graphs and numeric values, together with other images obtained by the ultrasound imaging apparatus, for example an anatomical image and a Doppler waveform, and thus created image is displayed on the monitor 14. There are various forms as the display image. Examples of display forms will be described later.

According to the present embodiment, diagnostic information into which three-dimensional effects are reflected can be provided, thereby enabling more precise understanding of cardiac blood flow dynamics, and the like.

<Second Embodiment>

The present embodiment is similar to the first embodiment, having the following processes; a process of calculating a blood flow velocity from echo signals by two methods, with regard to an identical position, a process of evaluating consistency of the blood flow velocities calculated by the two methods, a process of estimating three-dimensional effect of the blood flow in an imaging area, by using the consistency of the blood flow velocities, and a process of generating diagnostic information such as a pressure gradient between two points, a blood flow flux, and a tissue blood-flow interaction force, by using the three-dimensional effect of the blood flow being estimated.

The present embodiment features that the aforementioned processes are performed in each time phase, or in characteristic phases such as a contracting phase and a diastolic phase, by using cardiac cycle information inputted from the input unit 10 and image information obtained from a tomographic image former, thereby estimating the three-dimensional effect and generating diagnostic information. The cardiac cycle information is obtained on the basis of an ECG. It is also possible to utilize another information, such as a mitral-valve inflow velocity, a pulmonary-artery backflow velocity, a wall motion velocity, and a wall motion.

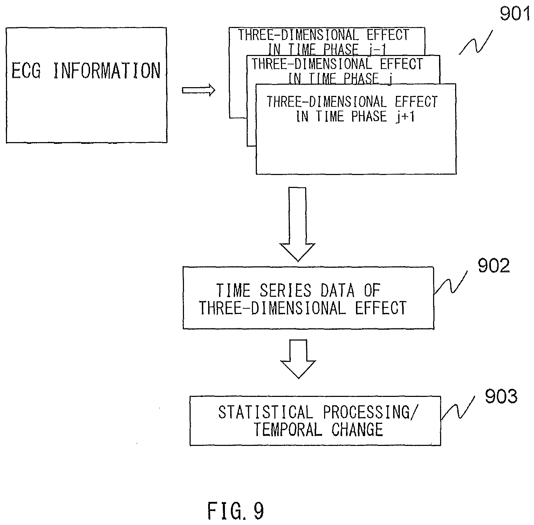

FIG. 9 illustrates one example of the process of the present embodiment. In the illustrated example, echo signals of each time phase are obtained with reference to the ECG, then blood flow vectors on an imaging cross section are calculated from those echo signals, and a three-dimensional effect is estimated. Accordingly, the three-dimensional effect 901 for each time phase is obtained. By using the three-dimensional effect 901 for each time phase, diagnostic information for each time phase is calculated. Various amounts calculated by the three-dimensional effect estimator 155 are the same as those of the first embodiment, including the diagnostic information such as the spatial rate of change of the blood flow velocity in the direction orthogonal to the imaging cross section, various amounts calculated therefrom, and further, the pressure gradient between two points, the flux of momentum or of kinetic energy, and the tissue blood-flow interaction force.

The diagnostic information 902 obtained in time series may be displayed, as it is, on the monitor 14 together with the ECG, thereby checking variations of the diagnostic information along with the cardiac cycle. In addition, progression of various amounts calculated by the three-dimensional effect estimator 155 may be displayed, and further, statistics 903 such as a maximum value, a minimum value, and an average value within a measurement period may be calculated, and further, a temporal change (a differential value), a value of integral, and the like, may be calculated. Instead of obtaining time-series data, specific time-phase data of interest may be selected, so as to generate diagnostic information therefrom.

According to the present embodiment, it is possible to provide diagnostic information into which the three-dimensional effect is reflected as information in association with a cardiac phase.

<Display examples>

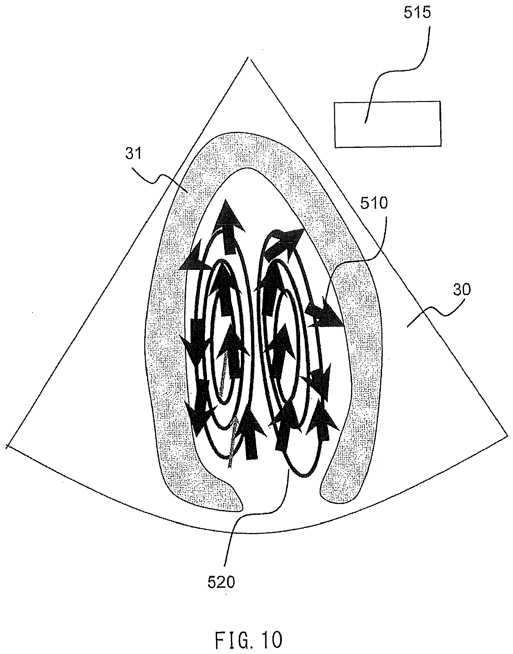

As described above, the ultrasound imaging apparatus of the present invention is able to provide various diagnostic information into which a three-dimensional effect is reflected, and the way of provision is not particularly limited. A typical provision method is to display the information in the form of display image on the monitor 14 of the apparatus. The display image generator 156 generates the display image. With reference to FIGs. 10 to FIG. 14, display examples (step S7 of the first embodiment) will be described in the following. It should be noted that the displaying methods are not limited to those examples, but various combinations thereof are also possible and elements may be omitted if they are not absolutely necessary.

FIG. 10 illustrates one example where the three-dimensional effect of the blood flow vectors is displayed. In the illustrated example, a monochrome tomographic image (cardiac muscle 31 is shown here) and blood-flow velocity vectors 510 are superimposed one on another. In addition, the three-dimensional effect (spatial rate of change of velocity in the direction orthogonal to the imaging area) 520 calculated in the step S5 is displayed in the form of contour. Furthermore, a three-dimensional effect monitor 515 is provided, and it is possible to display numerical values relating to the spatial rate of change 520 in the direction perpendicular to the imaging area, and other numerical values, or the like, such as an optional physical quantity and its residues, temporal change, and a distribution of pressure gradient on the three-dimensional effect monitor 515. A certain reference point may be provided for those various amounts, and a difference or a ratio with respect to the reference point may also be displayed.

Furthermore, a history of the three-dimensional effect of the blood-flow vectors is displayed, and at least one of a maximum value, a minimum value, an average value, and a value of variance within one heartbeat may also be displayed.

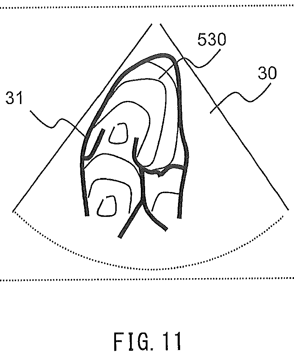

FIG. 11 shows an example where a pressure gradient distribution 530 is displayed on the screen. Also in this example, this spatial distribution of the pressure gradient 530 is displayed in such a manner as being superimposed on the monochrome tomographic image. It is further possible in this example to provide a certain reference point to display a difference or a ratio of the pressure gradient, and to display a history of the pressure gradient distribution to show at least one of a maximum value, a minimum value, an average value, and a value of variance within one heartbeat on the three-dimensional effect monitor (515: FIG. 10). The examiner may designate a desired portion within the image by manipulating the input part 10 such as a mouse, so as to display the designated portion on the screen.

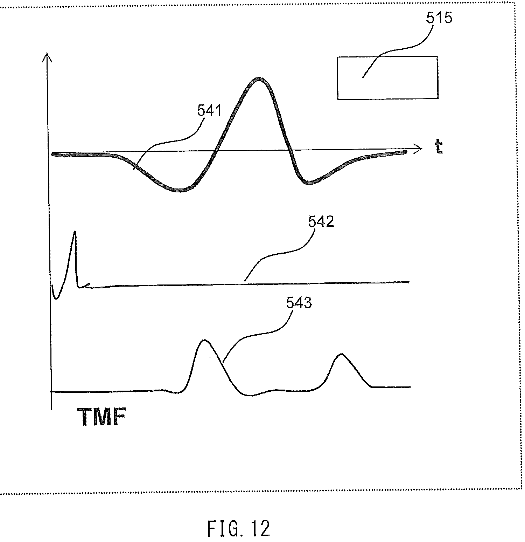

Similar to FIG. 11, the display example as shown in FIG. 12 indicates a pressure gradient between two points, and in this figure, the temporal change of the pressure gradient is shown in the form of graph 541. It is preferable that this graph 541 is shown together with any of temporal change of myocardial motion acquired by the tomographic image former, temporal change of the blood flow velocity acquired by the Doppler velocity operation part, and external ECG information. In the illustrated example, the ECG 542, the mitral-valve inflow waveform 543, and the graph 541 of pressure gradient between two points are shown. It is additionally possible to display temporal change of myocardial motion previously stored in the memory 157 and temporal change of the blood flow velocity acquired by the Doppler velocity operation part, in such a manner as cutting out the interval between R-wave and R-wave of the ECG (in sync with the interval of R-R). The myocardial motion may be M-mode information for acquiring temporal change at a portion desired by the examiner.

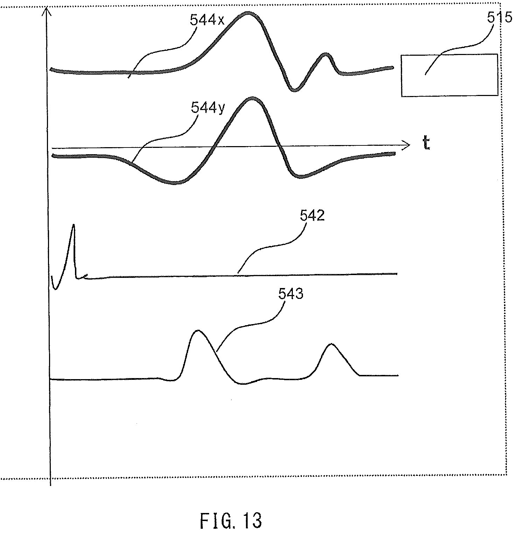

FIG. 13 shows an example that displays the tissue blood-flow interaction force generated by the diagnostic information generator 159. In the illustrated example, the x-direction component 544x of the tissue blood-flow interaction force and the y-direction component 544y of the tissue blood-flow interaction force are displayed in the form of graphs, each indicating the temporal change. Also in this case, the graphs are shown together with the ECG 542, the mitral-valve inflow waveform 543, and the like. It is additionally possible to display temporal change of myocardial motion previously stored in the memory 157 and temporal change of the blood flow velocity acquired by the Doppler velocity operation part, in such a manner as cutting out the interval between R-wave and R-wave of the ECG (in sync with the interval of R-R). The myocardial motion may be M-mode information for acquiring temporal change at a portion desired by the examiner.

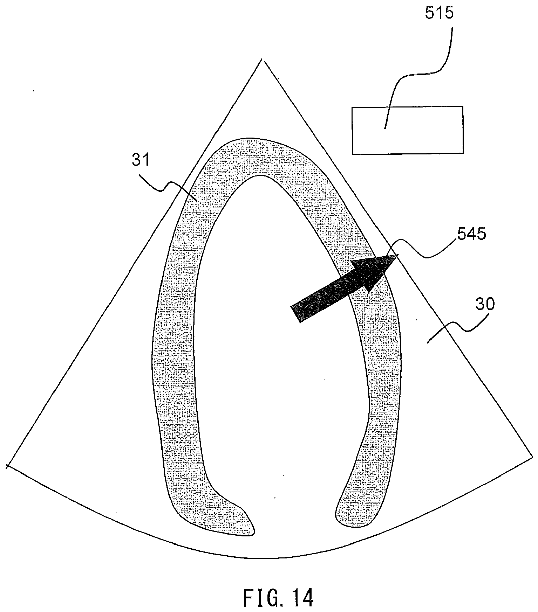

FIG. 14 shows an example that the tissue blood-flow interaction force is displayed in the form of vector 545, which is estimated on the basis of echo signals measured at a certain point of time, in such a manner as superimposed on the monochrome tomographic image. Also in this case, it is possible to display the tissue blood-flow interaction force being digitalized, other various amounts, and the like, on the three-dimensional effect monitor 515.

According to the present embodiment, various display forms are possible, and therefore, the examiner is allowed to receive information effective for diagnosis via the monitor. The displaying methods are not limited to those described examples. Various combinations thereof are available, or elements which are not absolutely necessary may be omitted. In addition, the monitor is not limited to the one provided in the ultrasound imaging apparatus of the present embodiment. It is of course possible to display data on other display device, by utilizing various publicly-known data transfer techniques.

**Industrial Applicability**

According to the present invention, the ultrasound diagnostic apparatus that is capable of estimating a blood-flow velocity vector from color Doppler imaging information, provides high-order diagnostic information with reflection of a blood-flow velocity component that is orthogonal to the imaging cross section. Accordingly, this may contribute to more precise diagnosis.

**Description of Symbols**

100 ...    ultrasound imaging apparatus
            1 main unit
            2 ultrasound probe
            10 input part
            11 controller
            12 ultrasound signal generator
            13 ultrasound receiving circuit
            14 monitor
            15 signal processor
            151 tomographic image former
            152 tissue velocity operation part
            153 blood-flow vector operation part
            154 Doppler velocity operation part (blood-flow velocity operation part)
            155 three-dimensional effect estimator
            158 spatial rate-of-change operation part
            159 diagnostic information generator

**Claims**

**1.** An ultrasound imaging apparatus comprising,

an ultrasound probe configured to transmit ultrasound waves to a test object and to receive echo signals reflected from the test object, and

a signal processor configured to process the echo signals received by the ultrasound probe, wherein, the signal processor estimates a three-dimensional effect of a blood flow velocity, from a difference between a first blood flow velocity estimated from the echo signals by a first method, and a second blood flow velocity estimated by a second method which is different from the first method, and generates diagnostic information into which the three-dimensional effect is reflected.

2. The ultrasound imaging apparatus according to claim 1, wherein,
the signal processor comprises,
a blood-flow velocity operation part configured to calculate the blood flow velocity from the echo signals, and
a three-dimensional effect estimator configured to estimate the three-dimensional effect on the basis of the blood flow velocity calculated by the blood-flow velocity operation part.

3. The ultrasound imaging apparatus according to claim 2, wherein,
the blood-flow velocity operation part comprises a tissue velocity operation part configured to calculate a tissue velocity of the test object and a Doppler velocity operation part, and calculates the blood flow velocity within an imaging plane, by using a tissue and blood-flow boundary velocity calculated by the tissue velocity operation part and a Doppler velocity calculated by the Doppler velocity operation part.

4. The ultrasound imaging apparatus according to claim 2, wherein,
the three-dimensional effect estimator comprises a spatial rate-of-change operation part configured to calculate as the three-dimensional effect, a spatial rate-of-change of the blood flow velocity in the direction orthogonal to an imaging plane, and various amounts derived therefrom.

5. The ultrasound imaging apparatus according to claim 4, wherein,
the spatial rate-of-change operation part has a distribution model of the spatial rate-of-change, and by using the distribution model, calculates a spatial rate-of-change of the blood flow velocity as to each plural points with different beam angles, on the basis of the difference between the blood flow velocities.

6. The ultrasound imaging apparatus according to claim 2, further comprising,
a diagnostic information generator configured to generate the diagnostic information, by using the three-dimensional effect estimated by the three-dimensional effect estimator.

7. The ultrasound imaging apparatus according to claim 6, wherein,
the diagnostic information generator generates a pressure gradient between two points, by using the three-dimensional effect estimated by the three-dimensional effect estimator.

8. The ultrasound imaging apparatus according to claim 6, wherein,
the diagnostic information generator calculates a remaining amount of a physical quantity in an imaging region, by using the three-dimensional effect estimated by the three-dimensional effect estimator.

9. The ultrasound imaging apparatus according to claim 8, wherein,
the physical quantity is at least one selected from mass, momentum, kinetic energy, a quantity of heat, and a concentration of a material.

10. The ultrasound imaging apparatus according to claim 8, wherein,
the physical quantity is kinetic energy in the imaging area, and
the diagnostic information generator calculates a tissue blood-flow interaction force as the diagnostic information, by using the remaining amount of the kinetic energy.

11. The ultrasound imaging apparatus according to claim 2, further comprising a monitor configured to display the diagnostic information.

12. The ultrasound imaging apparatus according to claim 11, wherein,
the monitor displays the diagnostic information in the form of a graph or numeric values, together with displaying a tomographic image and/or a blood flow vector.

**13.** The ultrasound imaging apparatus according to either of claim 11 and claim 12, wherein,
the monitor displays a temporal development of the diagnostic information.

**14.** The ultrasound imaging apparatus according to claim 1, further comprising an input part configured to input either of heartbeat information and ECG information externally, wherein,
the signal processor generates the diagnostic information of plural cardiac cycles, on the basis of either of the heartbeat information and the ECG information inputted from the input part.

**15.** An ultrasound imaging apparatus comprising,
an ultrasound probe configured to transmit ultrasound waves to a test object and to receive echo signals reflected from the test object, and
a signal processor configured to process the echo signals received by the ultrasound probe, wherein,
the signal processor comprises a blood-flow velocity operation part configured to calculate a blood flow velocity from the echo signals, and a spatial rate-of-change operation part configured to calculate a spatial rate-of-change of the blood flow velocity in a direction orthogonal to an imaging plane, on the basis of consistency of the blood flow velocities calculated by the blood-flow velocity operation part via different methods.

**16.** A method of generating diagnostic information of a test object, by using echo signals obtained by an ultrasound imaging apparatus, comprising,
calculating blood flow velocities from the echo signals via two methods, with regard to an identical position,
evaluating consistency of the blood flow velocities calculated via the two methods,
estimating a three-dimensional effect of a blood flow in an imaging region, by using the consistency of the blood flow velocities, and
generating diagnostic information including any of a pressure gradient between two points, a blood flow flux, and a tissue blood-flow interaction force, by using thus estimated three-dimensional effect of the blood flow.

FIG. 1

EP 3 111 850 A1

START

GENERATE TISSUE FORM IMAGE — S1

CALCULATE BLOOD FLOW VELOCITY — S3

CALCULATE TISSUE VELOCITY — S2

CALCULATE BLOOD FLOW VECTOR — S4

ESTIMATE BLOOD-FLOW VECTOR THREE-DIMENSIONAL EFFECT — S5

GENERATE DIAGNOSTIC INFORMATION — S6

DISPLAY DIAGNOSTIC INFORMATION — S7

END

FIG. 2

(a)

(b)

FIG. 3

FIG. 4

FIG. 5

COLOR DOPPLER IMAGE AND MYOCARDIAL TRACKING

FIG. 6

(a)

(b)

$F_{out}$

G - D

$F_{in}$

$\Delta z$

30

31

31

FIG. 7

31     31     31

521     522     523

(a)

31     31     31

523     524     525

(b)

FIG. 8

FIG. 9

FIG. 10

FIG. 11

FIG. 12

FIG. 13

FIG. 14

**INTERNATIONAL SEARCH REPORT**

| International application No. |
| --- |
| PCT/JP2015/051281 |

| A. CLASSIFICATION OF SUBJECT MATTER |
| --- |
| *A61B8/06*(2006.01)i, *A61B8/14*(2006.01)i |

According to International Patent Classification (IPC) or to both national classification and IPC

| B. FIELDS SEARCHED |
| --- |

Minimum documentation searched (classification system followed by classification symbols)
A61B8/06, A61B8/14

Documentation searched other than minimum documentation to the extent that such documents are included in the fields searched

| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)
JSTPlus(JDreamIII), JMEDPlus(JDreamIII)

| C. DOCUMENTS CONSIDERED TO BE RELEVANT |
| --- |

| Category* | Citation of document, with indication, where appropriate, of the relevant passages | Relevant to claim No. |
| --- | --- | --- |
| A | WO 2013/157553 A1 (Hitachi Aloka Medical, Ltd.), 24 October 2013 (24.10.2013), entire text; all drawings & CN 104220005 A | 1–16 |
| A | JP 2013-176492 A (Hitachi Aloka Medical, Ltd.), 09 September 2013 (09.09.2013), entire text; all drawings (Family: none) | 1–16 |
| A | JP 2008-80106 A (Toshiba Corp., Toshiba Medical Systems Corp.), 10 April 2008 (10.04.2008), entire text; all drawings & US 2008/0051661 A1 & JP 5231768 B2 | 1–16 |

☒ Further documents are listed in the continuation of Box C.     ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 25 March 2015 (25.03.15) | 07 April 2015 (07.04.15) |

| Name and mailing address of the ISA/ | Authorized officer |
| --- | --- |
| Japan Patent Office 3-4-3,Kasumigaseki,Chiyoda-ku, Tokyo 100-8915,Japan | |
| | Telephone No. |

Form PCT/ISA/210 (second sheet) (July 2009)

**INTERNATIONAL SEARCH REPORT**

International application No.

PCT/JP2015/051281

C (Continuation).   DOCUMENTS CONSIDERED TO BE RELEVANT

| Category* | Citation of document, with indication, where appropriate, of the relevant passages | Relevant to claim No. |
|---|---|---|
| A | Takashi OKADA, Keiichi ITAYA, Kagami MIYAJI, "4. Ketsuryu no Sokudo Vector Hyoji Vector Flow Mapping(VFM)", INNERVISION, 25 February 2014 (25.02.2014), vol.29, no.3, page 70 | 1-16 |

Form PCT/ISA/210 (continuation of second sheet) (July 2009)

**EP 3 111 850 A1**

**Patent documents cited in the description**

- JP 2000342585 A

- JP 11313824 A